# EUROPÄISCHE PATENTSCHRIFT

(11) **EP 1 570 796 B1**
(45) Veröffentlichungstag und Bekanntmachung des Hinweises auf die Patenterteilung: **16.05.2007**
(21) Anmeldenummer: 05004677.0
(22) Anmeldetag: 03.03.2005
(51) Int. Cl.: A61B 17/70

(54) **Knochenverankerungselement und Stabilisierungseinrichtung mit einem solchen Knochenverankerungselement**
Bone fixing element and stabilising device comprising one such bone fixing element
Elément d'ancrage osseux et dispositif de stabilisation comprenant un tel élément d'ancrage osseux

(30) Priorität: 03.03.2004 DE 102004010382; 03.03.2004 US 550009 P
(43) Veröffentlichungstag der Anmeldung: 07.09.2005
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE); Matthis, Wilfried, 79367 Weisweil (DE)
(74) Vertreter: Hofer, Dorothea

(56) Entgegenhaltungen:
- EP-A- 1 273 269
- WO-A-98/34554
- US-A- 6 022 350

## Beschreibung

Die Erfindung betrifft ein Knochenverankerungselement zur Verankerung in einem Knochen oder einem Wirbel und eine Stabilisierungseinrichtung mit einem solchen Knochenverankerungselement.

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder einen Stab verbundenen Knochenschrauben bestehen. Derartige starre Systeme erlauben keine Bewegung der relativ zueinander fixierten Knochenteile oder Wirbel.

Für bestimmte Indikationen ist jedoch eine dynamische Stabilisierung wünschenswert, bei der die zu stabilisierenden Knochenteile oder Wirbel eine kontrollierte begrenzte Bewegung zueinander ausführen können. Eine Realisierungsmöglichkeit für eine dynamische Stabilisierungseinrichtung besteht in der Verwendung eines beweglichen Elements anstelle eines die Knochenverankerungselemente verbindenden starren Stabes, wie es beispielsweise in der EP 0 669 109 B1 oder in der US 2003/0109880 A1 beschrieben ist.

Bei diesen bekannten Systemen kann häufiger das Problem auftreten, dass eine Übertragung der aus der Bewegung resultierenden Momente auf den im Knochen oder Wirbel verankerten Schaft der Verankerungselemente stattfindet, was aufgrund der dauernden zyklischen Teilbelastung des Knochenverankerungselements zur Lockerung des Schafts in dem umgebenden Knochenmaterial und damit zum Lösen der Verankerung führen kann.

Es besteht daher das Bedürfnis, die Bewegung der Knochen bzw. Wirbel von der Schaftverankerung weitgehend abzukoppeln, um der Forderung gerecht zu werden, dass das Implantat oft ein Leben lang sicher im Körper des Patienten verankert bleiben muss. Ferner soll aber bei einer Entkopplung der Bewegung der Knochen bzw. Wirbel von der Schaftverankerung trotzdem die Möglichkeit bestehen, das Verankerungselement relativ zu einem mehrere solcher Elemente verbindenden Stab oder zu einer solchen Platte optimal positionieren zu können, wie es z.B. von den herkömmlichen Polyaxialschrauben bekannt ist.

Die US 6,022,350 offenbart eine Knochenbefestigungsvorrichtung mit einem länglichen Verbindungsmittel, das eine durch eine Öffnung in dem Verbindungsmittel hindurch geführte Knochenbefestigungsschraube aufnimmt. In dem Boden des Verbindungsmittels ist eine Lagerungsfläche mit im Wesentlichen kreisförmigem Querschnitt vorgesehen und der Kopf der Schraube beinhaltet eine im Wesentlichen kugelförmige Oberfläche, die sich an der Lagerungsfläche abstützt. Bei einer Ausführungsform sind in der Öffnung in dem Verbindungsmittel ferner zwei O-Ringe aus z.B. Silikon vorgesehen.

Aus der US 5,474,555 ist ferner ein Knochenverankerungselement in Form einer Polyaxial-Knochenschraube mit einem Schraubenelement und einem Aufnahmeteil zur Verbindung mit einem Stab bekannt, bei der das im Knochen zu verankernde Schraubenelement mit dem Aufnahmeteil derart verbunden ist, daß Schwenkbewegungen zwischen dem Schraubenelement und dem Aufnahmeteil möglich sind. Die beschriebene Lösung erlaubt jedoch keine Stabilisierung der Knochenteile untereinander mit einer kontrollierten Teilbewegung.

Es ist Aufgabe der Erfindung ein Knochenverankerungselement und eine Stabilisierungseinrichtung für Knochen bzw. Wirbel mit einem solchen Knochenverankerungselement bereitzustellen, das bzw. die eine kontrollierte Teilbewegung der zu stabilisierenden Knochenteile oder Wirbel bei gleichzeitig hoher Sicherheit der Verankerung erlaubt und dabei optimal positionierbar ist.

Die Aufgabe wird gelöst durch ein Knochenverankerungselement zur Verankerung in einem Knochen oder in einem Wirbel nach Patentanspruch 1 und durch eine Stabilisierungseinrichtung mit einem solchen Knochenverankerungselement nach Patentanspruch 15.

Weiterbildungen der Erfindung sind in den Unteransprüchen angegeben.

Die Erfindung weist den Vorteil auf, daß das Knochenverankerungselement aufgrund seiner elastischen Verbindung von erstem und zweitem Kopfteil axiale Kräfte in Richtung seiner Schaftachse, Biegekräfte und Torsionskräfte aufnimmt. Damit wird eine durch die rücktreibende Kraft begrenzte Bewegung des Knochenteils oder Wirbels, in dem es verankert ist, erlaubt und die bei der Bewegung auf den Schaft wirkenden Momente werden reduziert, so dass ein Lösen der Verbindung durch dauernde zyklische Teilbelastung der Verankerung unterbleibt.

Die Erfindung weist weiterhin den Vorteil auf, dass eine gewünschte Ruhewinkelstellung zwischen dem Schaft und dem Aufnahmeteil wie bei einer herkömmlichen Polyaxialschraube justierbar ist.

Die elastischen Eigenschaften des Knochenverankerungselements können bei der Herstellung auf einfache Art durch Ändern der Dimensionen von erstem Kopfteil, zweitem Kopfteil und elastischem Druckelement und/oder des verwendeten Materials für das elastische Druckelement verwirklicht werden. Das Knochenverankerungselement ist mit den bekannten Verbindungselementen wie Platten und Stäben verwendbar. Somit kann eine dynamische Stabilisierungseinrichtung mit einer gewünschten Bewegungsbegrenzung durch Auswahl von Knochenverankerungselementen mit geeigneten elastischen Eigenschaften unter Verwendung von herkömmlichen Platten oder Stäben bereitgestellt werden.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine teilgeschnittene Darstellung eines Knochenverankerungselements nach einer ersten Ausführungsform;
- Fig. 2: eine teilgeschnittene Explosionsdarstellung des Knochenverankerungselements der ersten Ausführungsform;
- Fig. 3a bis 3c: teilgeschnittene Darstellungen, die eine elastische Auslenkung des Knochenverankerungselements der ersten Ausführungsform aus der Ruhewinkelstellung schematisch darstellen.
- Fig. 4: eine teilgeschnittene Darstellung eines Knochenverankerungselements nach einer zweiten Ausführungsform;
- Fig. 5: eine teilgeschnittene Darstellung eines Knochenverankerungselements nach einer dritten Ausführungsform; und
- Fig. 6: eine teilgeschnittene Darstellung eines Knochenverankerungselements nach einer vierten Ausführungsform.

### Erste Ausführungsform

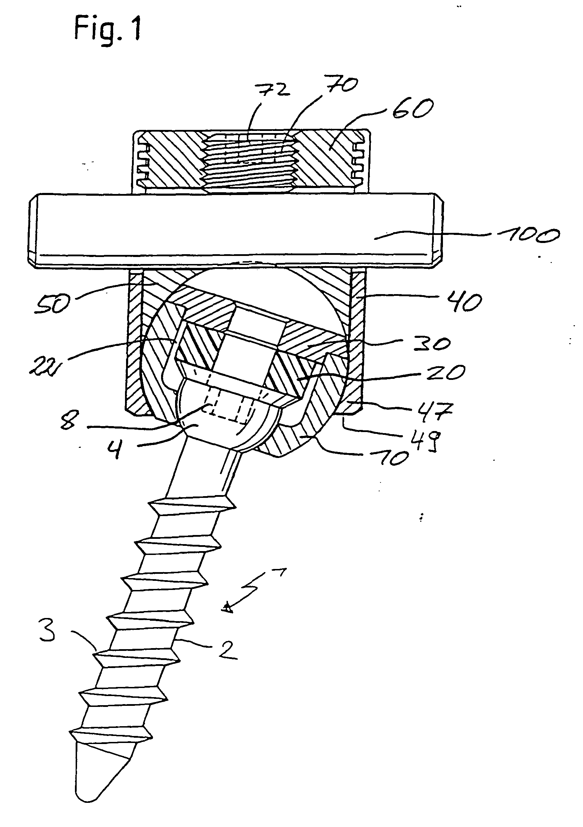

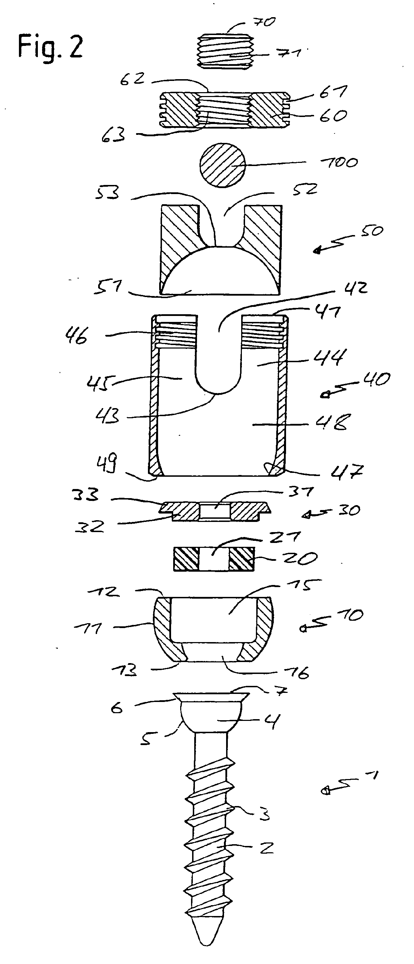

Ein Knochenverankerungselement nach der in den Fig. 1 und 2 gezeigten ersten Ausführungsform ist als Polyaxial-Knochenschraube zum Verbinden mit einem Stab 100 ausgebildet. Die Polyaxial-Knochenschraube weist ein im Knochen zu verankerndes Schraubenelement 1 mit einem Schaft 2 mit einem Knochengewinde 3 und mit einem mit dem Schaft 2 in diesem Ausführungsbeispiel einstückig verbundenen ersten Kopfteil 4 auf. Desweiteren weist die Polyaxial-Knochenschraube ein hülsenförmiges zweites Kopfteil 10 zur Aufnahme des ersten Kopfteils 4 und ein Aufnahmeteil 40 zur Aufnahme des zweiten Kopfteiles 10, eines auf dieses einwirkenden zweiten Druckelements 50 und des Stabes 100, sowie eine erste und eine zweite Sicherungsschraube 60, 70 auf.

Das erste Kopfteil 4 des Schraubenelements 1 weist angrenzend an den Schaft 2 einen kugelsegmentförmigen Abschnitt 5 mit einem Durchmesser auf, der größer ist als der Schaftdurchmesser. Auf der dem Schaft 3 abgewandten Seite ist ein verbreiterter Rand 6 bzw. Kragen vorgesehen, wodurch eine kreisförmige Fläche 7 gebildet ist, die im Wesentlichen glatt ist und in der sich koaxial zur Schaftachse eine Ausnehmung 8 zum Eingreifen mit einem Schraubwerkzeug befindet. In Fig.1 ist eine sechskantförmige Ausnehmung 8 für einen Inbusschlüssel dargestellt.

Es ist jedoch auch eine andere Ausnehmung, wie z.B. ein Kreuzschlitz, zum Eingreifen mit einem Einschraubwerkzeug möglich.

Das hülsenförmige zweite Kopfteil 10 ist kugelsegmentförmig mit einer sphärischen Außenfläche 11 und zwei gegenüberliegenden ebenen Enden 12, 13 ausgebildet. Von dem einen Ende 12 erstreckt sich eine koaxiale erste Bohrung 15 mit einem Durchmesser, der größer ist als der Außendurchmesser des verbreiterten Rands 6 des Schraubenelementes 1. Angrenzend an die erste Bohrung 15 erstreckt sich eine koaxiale zweite Bohrung 16 deren Durchmesser angrenzend an das zweite Ende 13 kleiner ist als der Kugeldurchmesser des ersten Kopfteils 4 aber größer als der Durchmesser des Schafts 2, so daß dieser hindurchgeführt werden kann. Die zweite Bohrung 16 erweitert sich ferner ausgehend vom zweiten Ende 13 bis zur ersten Bohrung 15 hin sphärisch mit einem Kugelradius, der so bemessen ist, daß das zweite Kopfteil 4 daran anliegt. Angrenzend an die erste Bohrung 15 ist der Durchmesser der zweiten Bohrung kleiner als der Kugelradius des Kopfteils 4.

Das Schraubenelement 1 ist derart in das zweite Kopfteil 10 eingesetzt, dass der Schaft 2 durch das Ende 13 angrenzend an die Bohrung 16 hindurchgeführt ist und der kugelsegmentförmige Abschnitt 5 des ersten Kopfteils 4 in der zweiten Bohrung 16 des zweiten Kopfteils 10 gehalten ist.

Auf der von dem verbreiterten Rand 6 des ersten Kopfteils 4 gebildeten Fläche 7 ist ein elastisches erstes Druckelement 20 angeordnet, das bevorzugt aus einem Elastomer ausgebildet ist. Das erste Druckelement 20 weist die Form einer Scheibe mit kreisförmigem Querschnitt und einer vorbestimmten Dicke auf und besitzt eine koaxiale Bohrung 21, die ein Hindurchgreifen mit dem Schraubwerkzeug in die Ausnehmung 8 der von dem verbreiterten Rand 6 gebildeten Fläche 7 ermöglicht. Der Außendurchmesser des ersten Druckelements 20 ist so gewählt, dass es in die erste Bohrung 15 des zweiten Kopfteils 10 einführbar ist. Vorzugsweise ist zwischen Druckelement 20 und der Seitenwand der Bohrung ein Freiraum 22 vorgesehen, so dass das Druckelement bei elastischer Deformation ausweichen kann.

Auf der dem Rand 6 des ersten Kopfteils 4 abgewandten Seite des ersten Druckelements 20 ist eine starre Befestigungskappe 30 angeordnet. Die Befestigungskappe 30 weist die Form einer kreisförmigen Scheibe mit einer koaxialen Bohrung 31 auf, die ein Hindurchgreifen mit dem oben genannten Schraubwerkzeug ermöglicht.

Der Außendurchmesser der Befestigungskappe 30 entspricht in einem dem ersten Druckelement 20 zugewandten Abschnitt 32 dem Innendurchmesser der ersten Bohrung 15 in dem zweiten Kopfteil 10 bzw. ist um soviel kleiner, dass ein Einsetzen und Fixieren z.B. mit Presspassung in dem zweiten Kopfteil 10 ermöglicht ist.

In einem in eingesetztem Zustand dem ersten Druckelement 20 abgewandten Abschnitt 33 weist die Befestigungskappe 30 einen verbreiterten Außendurchmesser auf, der beim Einsetzen in das zweite Kopfteil 10 durch Zusammenwirken mit dem ersten Ende 12 des zweiten Kopfteils 10 einen Anschlag bildet. Die Außenfläche des Abschnitts 33 ist derart sphärisch ausgebildet, dass sie im eingesetzten Zustand der Befestigungskappe 30 eine Fortführung der sphärischen Außenfläche 11 des zweiten Kopfteils 10 bildet.

Die Dicke des ersten Druckelements 20 ist so gewählt, dass es, wenn die Befestigungskappe 30 in das zweite Kopfteil eingesetzt ist, unter Vorspannung gesetzt ist und Druck auf die von dem verbreiterten Rand 6 des ersten Kopfteils 4 gebildete Fläche 7 ausübt, so dass das erste Kopfteil 4 gegen die zweite Bohrung 16 des zweiten Kopfteils 10 gedrückt wird und das Schraubenelement 1 elastisch in dem zweiten Kopfteil 10 gehalten ist. Vorteilhafterweise ist die Lagerung des ersten Kopfteils 4 in der zweiten Bohrung 16 durch Einbringen einer speziellen Gleitbeschichtung, einer Gleitfolie oder eines Gleitringes im Hinblick auf minimale zwischen dem kugelsegmentförmigen Abschnitt 5 und der zweiten Bohrung 16 wirkende Reibungskräfte optimiert.

Das Aufnahmeteil 40 ist im Wesentlichen zylindrisch ausgebildet. Ausgehend von seinem ersten Ende 41 ist in dem Aufnahmeteil 40 eine koaxiale erste Bohrung 48 vorgesehen, deren Durchmesser größer ist, als der Außendurchmesser des zweiten Kopfteils 10. Angrenzend an das andere Ende 49 des Aufnahmeteils 40 ist eine koaxiale zweite Bohrung 47 vorgesehen, deren Durchmesser sich vom Durchmesser der ersten Bohrung 48 gegen das zweite Ende 49 hin sphärisch verjüngt. Dadurch ist das zweite Kopfteil 10 in dem Aufnahmeteil 40 gehalten und um einen vorbestimmten Winkelbereich schwenkbar.

Ausgehend von seinem einen ersten Ende 41 ist ferner eine Ausnehmung 42 mit einem U-förmigen Querschnitt vorgesehen, die gerade so groß bemessen ist, dass der Stab 100 einlegbar ist. Durch die U-förmige Ausnehmung 42 sind zwei freie Schenkel 44, 45 gebildet, die angrenzend an ihr freies Ende ein Innengewinde 46 aufweisen, welches mit einem entsprechenden Außengewinde 61 der zwischen die Schenkel 44, 45 einzuschraubenden ersten Sicherungsschraube 60 zusammenwirkt.

Auf der der zweiten Bohrung 47 abgewandten Seite befindet sich in dem Aufnahmeteil 40 ein auf das zweite Kopfteil 10 einwirkendes zweites Druckelement 50. Das zweite Druckelement 50 ist im Wesentlichen zylindersymmetrisch mit einem Außendurchmesser, der kleiner ist, als der Innendurchmesser der ersten Bohrung 48, dass das Druckelement in die erste Bohrung 48 einsetzbar ist. Die dem zweiten Kopfteil 10 zugewandte Stirnseite 51 des zweiten Druckelements 50 ist konkav sphärisch ausgebildet, wobei der Radius dem Radius der sphärischen Außenfläche 11 des zweiten Kopfteils 10 und der daran befestigten Befestigungskappe 30 entspricht. Auf der dem zweiten Kopfteil 10 abgewandten Seite weist das zweite Druckelement 50 eine Ausnehmung 52 mit U-förmigem Querschnitt auf, deren Grund 53 einen Radius aufweist, der etwas größer als der Radius des Stabes 100 ist und in die der Stab 100 eingreift.

Das zweite Druckelement 50 weist ferner eine koaxiale Bohrung zum Hindurchgreifen mit einem Schraubwerkzeug auf.

In Richtung der Zylinderachse des Aufnahmeteils 40 ist die Höhe des zweiten Druckelements 50 gerade so gewählt, dass durch Anziehen der ersten Sicherungsschraube 60 derart Druck auf das zweite Druckelement 50 ausgeübt werden kann, dass das zweite Kopfteil 10 in dem Aufnahmeteil 40 in einer zu dem Zeitpunkt des Anziehens der Sicherungsschraube 60 eingestellten Winkelstellung vorfixierbar ist, die nachfolgend als Ruhewinkelstellung bezeichnet ist.

Mit dieser Anordnung wird erreicht, dass das zweite Kopfteil 10 relativ zu dem Aufnahmeteil 40 ausgerichtet und anschlie-ßend in dieser Ausrichtung vorfixiert werden kann.

Die erste Sicherungsschraube 60 weist eine Bohrung 62 auf, die mit einem Innengewinde 63 versehen ist, das mit einem Außengewinde 71 der zweiten Sicherungsschraube 70 zusammenwirkt. Desweiteren weist die Sicherungsschraube 60 auf ihrer dem Druckelement 50 abgewandten Seite nicht dargestellte Ausnehmungen zum Eingreifen mit einem Schraubwerkzeug auf.

Die zweite Sicherungsschraube 70 weist an ihrer Oberseite eine Ausnehmung 72 zum Eingreifen mit einem Schraubwerkzeug auf. In Fig.1 ist eine sechskantförmige Ausnehmung 72 für einen Inbusschlüssel dargestellt. Es ist jedoch auch eine andere Ausnehmung, wie z.B. ein Kreuzschlitz, zum Eingreifen mit einem Einschraubwerkzeug möglich.

Durch Anziehen der zweiten Sicherungsschraube 70 wird der Stab 100 gegen den Grund 53 der Ausnehmung 52 gedrückt und damit Druck auf das zweite Druckelement 50 und damit auf das zweite Kopfteil 10 ausgeübt, um ein optimales Fixieren der Ruhewinkelstellung zu erzielen. Der Stab 100 wird dabei durch die zweite Sicherungsschraube 70 und den Grund 53 in der U-förmigen Ausnehmung 42 des Aufnahmeteils fixiert.

Im Betrieb wird das Schraubenelement 1 zuerst mit dem Schaft 2 durch die Bohrungen 15, 16 in dem zweiten Kopfteil 10 hindurchgeführt, sodass das erste Kopfteil 4 in der zweiten Bohrung 16 gehalten wird. Anschließend wird das erste Druckelement 20 in das zweite Kopfteil 10 eingeführt und durch Einsetzen der Befestigungskappe 30 in das zweite Kopfteil 10 gegen die Fläche 7 des ersten Kopfteils 4 gedrückt und in diesem vorgespannten Zustand über die eingesetzte Befestigungskappe 30 gehalten. Dann wird das aus Schraube 1 und zweitem Kopfteil 10 gebildete Element in das Aufnahmeteil 40 eingebracht und anschließend das zweite Druckelement 50 in das Aufnahmeteil 40 eingesetzt. Dann wird das Schraubenelement 1 durch Hindurchgreifen durch die koaxialen Bohrungen des zweiten Druckelements 50, der Befestigungskappe 30 und des ersten Druckelements 20 und Eingreifen in die Ausnehmung 8 des Schraubenelements mit einem Schraubendreher in den Knochen oder Wirbel eingeschraubt. Anschließend wird der Stab eingelegt, wobei die Stellung des Aufnahmeteils 40 relativ zu dem zweiten Kopfteil 10 justiert wird. Diese Stellung, die die gewünschte Ruhewinkelstellung ist, wird durch die erste Sicherungsschraube 60, die auf das zweite Druckelement 50 drückt, vorfixiert.

Bei dem Vorfixieren der Ruhewinkelstellung wird die Befestigungskappe 30 durch Zusammenwirken ihrer sphärischen Außenfläche mit der sphärischen Ausnehmung der Stirnseite 51 des zweiten Druckelements 50 gegen das erste Druckelement 20 gedrückt und somit gegen Lösen gesichert.

Der Stab 100 wird anschließend durch Einschrauben der zweiten Sicherungsschraube 70 gegen den Grund 53 gedrückt und dadurch fixiert. Gleichzeitig wird durch das Anziehen der zweiten Sicherungsschraube 70 über den Stab 100 auf das zweite Druckelement 50 und damit auf das zweite Kopfteil 10 Druck ausgeübt, sodass damit eine zusätzliche Fixierung der Ruhewinkelstellung erzielt wird.

Das Vorsehen der beiden Sicherungsschrauben 60, 70 führt dazu, dass das zweite Kopfteil 10 und der Stab 100 unabhängig voneinander fixierbar sind.

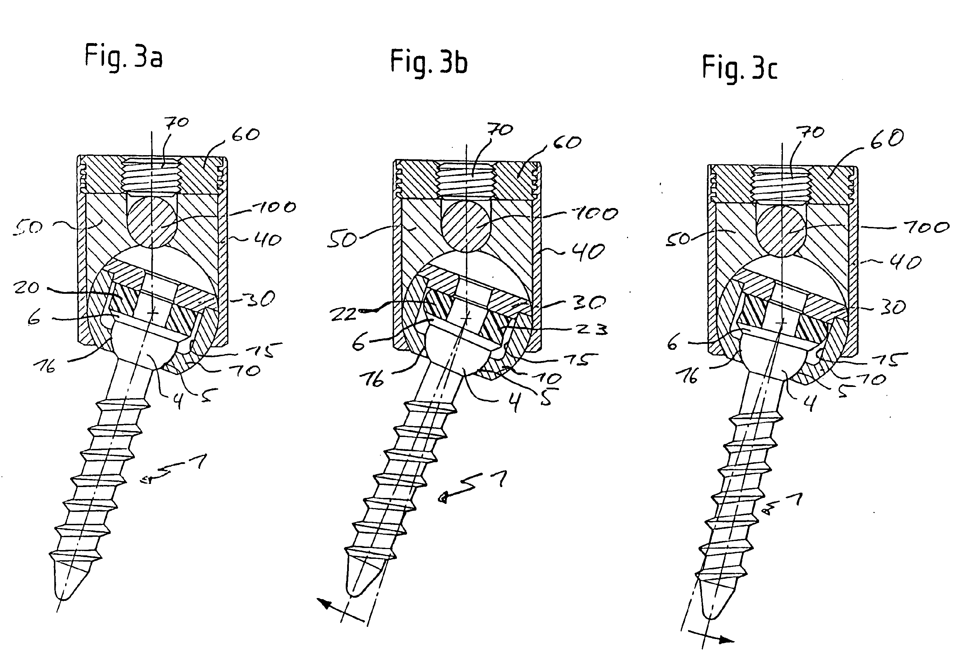

In den Fig. 3a bis 3c ist schematisch die Auslenkbarkeit des Schraubenelementes 1 relativ zu dem zweiten Kopfteil 10 dargestellt. In Fig. 3a ist eine voreingestellte Ruhewinkelstellung gezeigt, die Fig. 3b und 3c zeigen jeweils eine Auslenkung aus dieser Ruhewinkelstellung in unterschiedliche Richtungen, wobei der Pfeil die Auslenkungsrichtung bezeichnet.

Wie in Fig. 3a gezeigt ist, weist das erste Druckelement 20 in der Ruhewinkelstellung eine gleichmäßige Dicke auf. In Fig. 3b ist schematisch dargestellt, dass ein Auslenken des Schraubenelements dazu führt, dass eine Seite 22 des Druckelements komprimiert wird, wohingegen die gegenüberliegende Seite 23 entspannt wird. Das Verformen des elastischen ersten Druckelementes 20 führt zum Aufbau internen Spannungen in diesem, die eine der Auslenkung entgegenwirkende rücktreibende Kraft ausüben, sodass das Schraubenelement 1 in die Ruhewinkelstellung zurückgedrängt wird.

Die Größe der rücktreibenden Kraft kann dabei zum Einen durch die Dicke des ersten Druckelements 20 beeinflusst werden, die die Vorspannung des ersten Druckelements 20 im eingebauten Zustand bestimmt. Desweiteren kann die Größe der rücktreibenden Kraft auch durch die Materialwahl für das erste Druckelement 20, wobei unterschiedlichste Elastizitäten und Kompressibilitäten möglich sind, und durch die genaue Formgebung des ersten Druckelements 20 eingestellt werden, so dass eine gewünschte Steifigkeit des Knochenverankerungselements gegenüber axialen Kräften, Biegekräften und Torsionskräften erzielt werden kann.

Bei der Auslenkung aus der Ruhewinkelstellung begrenzt das Verhältnis des Durchmessers des Rands 6 des Schraubenelementes 1 zu dem Durchmesser der ersten Bohrung 15 in dem zweiten Kopfteil 10 die Auslenkbarkeit. Durch unterschiedliche Größenverhältnisse zwischen dem Durchmesser des Randes 6 und dem Innendurchmesser der Bohrung 15 kann somit die Auslenkbarkeit variiert werden. Z.B. können durch Wahl von Schrauben 1 mit unterschiedlich breiten Rändern 6, die in ein zweites Kopfteil mit vorgegebenen Innendurchmesser der Bohrung 15 einsetzbar sind, somit Verankerungselemente mit unterschiedlicher Auslenkbarkeit bereitgestellt werden.

Ein besonderer Vorteil dieser Ausbildung eines Knochenverankerungselements ist die Entkopplung der elastischen Auslenkung des Schraubenelements 1 von der Einstellmöglichkeit der Ruhewinkelstellung, was durch die Kombination von erstem Kopfteil 4, zweitem Kopfteil 10 und Aufnahmeteil 40 erreicht wird.

Eine erfindungsgemäße Stabilisierungseinrichtung umfasst wenigstens ein Knochenverankerungselement wie oben beschrieben in Kombination mit einem Stab und ein zweites Knochenverankerungselement, das bevorzugt ebenfalls wie oben beschrieben ausgebildet ist.

Bei einer Bewegung des Knochens bzw. Wirbels aus der zu stabilisierenden Ruhelage heraus wird über das elastische erste Druckelement 20 eine rücktreibende Kraft auf den Knochen bzw. Wirbel ausgeübt, die ihn wieder in die Ruhelage bringt und somit die Bewegung limitiert. Dadurch wird ein über die Bewegung des Knochens oder des Wirbels auf den Schraubenschaft wirkendes Biegemoment abgefangen und es kommt nicht zu einer Lockerung der Knochenverankerung.

### Zweite Ausführungsform

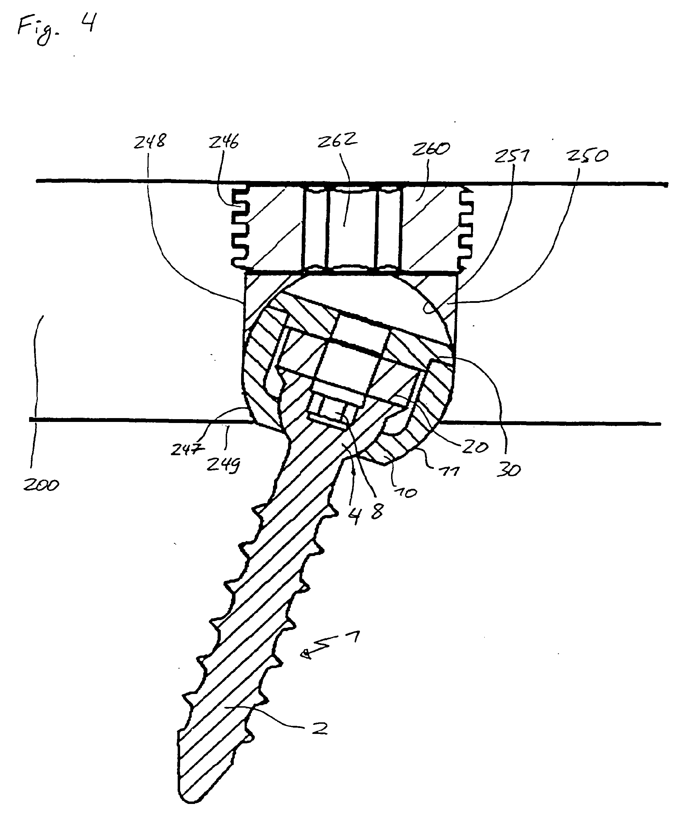

Bei einem Knochenverankerungselement der zweiten Ausführungsform, die in Fig. 4 dargestellt ist, ist das Knochenverankerungselement derart ausgebildet, dass nicht das Befestigen eines Stabes, sondern einer Platte vorgesehen ist. Bei dieser Ausführungsform entspricht der Aufbau von erstem Schraubenelement 1, erstem Druckelement 20, Befestigungskappe 30 und zweitem Kopfteil 10 dem Aufbau der ersten Ausführungsform, weshalb für diese Komponenten dieselben Bezugszeichen verwendet werden und eine erneute Beschreibung unterbleibt.

Der wesentliche Unterschied der zweiten Ausführungsform gegenüber der ersten Ausführungsform besteht darin, dass das Aufnahmeteil durch die zu befestigende Platte 200 gebildet wird.

Die Platte 200 weist eine erste Bohrung 248 auf, die von einer ersten Seite der Platte in die Platte hineinreicht und deren Durchmesser größer ist, als der Außendurchmesser des zweiten Kopfteils 10. Die erste Bohrung 248 weist in dem der ersten Seite der Platte zugewandten Bereich desweiteren ein Innengewinde 246 auf. Angrenzend an die andere Seite 249 der Platte ist koaxial zur ersten Bohrung 248 und verbunden mit dieser eine zweite Bohrung 247 vorgesehen, deren Durchmesser sich vom Durchmesser der ersten Bohrung 248 gegen die zweite Seite 249 hin sphärisch verjüngt. Dadurch ist das zweite Kopfteil 10 in der Platte 200 gehalten und um einen vorbestimmten Winkelbereich schwenkbar, wie bei der ersten Ausführungsform.

Auf der der zweiten Bohrung 247 abgewandten Seite befindet sich in der ersten Bohrung 248 ein auf das zweite Kopfteil 10 einwirkendes zweites Druckelement 250. Das zweite Druckelement 250 ist im Wesentlichen zylindersymmetrisch mit einem Außendurchmesser, der kleiner ist, als der Innendurchmesser der ersten Bohrung 248, sodass das Druckelement in die erste Bohrung 248 einsetzbar ist. Die dem zweiten Kopfteil 10 zugewandte Stirnseite 251 des zweiten Druckelements 250 ist konkav sphärisch ausgebildet, wobei der Radius dem Radius der sphärischen Außenfläche 11 des zweiten Kopfteils 10 und der daran befestigten Befestigungskappe 30 entspricht. Das zweite Druckelement 250 weist ferner eine koaxiale Bohrung zum Hindurchgreifen mit einem Schraubwerkzeug auf.

In Richtung der Bohrung in der Platte ist die Höhe des zweiten Druckelements 250 gerade so gewählt, dass durch Anziehen einer Sicherungsschraube 260, die in das Innengewinde 246 der Bohrung 248 einschraubbar ist, derart Druck auf das zweite Druckelement 250 ausgeübt werden kann, dass das zweite Kopfteil 10 in der Platte in einer zu dem Zeitpunkt des Anziehens der Sicherungsschraube 260 eingestellten Winkelstellung fixierbar ist, die nachfolgend als Ruhewinkelstellung bezeichnet ist, und die Platte dabei gleichzeitig mit dem Schraubenelement 1 verbunden wird.

Mit dieser Anordnung wird erreicht, dass das zweite Kopfteil 10 relativ zu der Platte 200 ausgerichtet und anschließend in dieser Ausrichtung vorfixiert werden kann.

Die Sicherungsschraube 260 weist eine koaxiale Bohrung 262 zum Eingreifen mit einem Schraubwerkzeug auf. In Fig. 4 ist eine sechskantförmige Bohrung 262 für einen Inbusschlüssel dargestellt. Es ist jedoch auch eine andere Formgebung zum Eingreifen mit einem Einschraubwerkzeug möglich.

Die koaxiale Bohrung 262 weist einen solchen Querschnitt auf, dass ein Hindurchgreifen mit dem Schraubwerkzeug, für das die Ausnehmung 8 des ersten Kopfteils vorgesehen ist, ermöglicht ist.

Diese Ausführungsform weist den besonderen Vorteil auf, dass der gesamte Aufbau vormontiert werden kann. Im Betrieb ist zuerst nur die Sicherungsschraube 260 nicht angezogen. Das Schraubenelement kann in dem vormontierten Zustand in den Knochen oder Wirbel eingeschraubt werden, indem mit dem Schraubwerkzeug durch die konzentrischen Bohrungen in dem ersten Druckelement 20, der Befestigungskappe 30, des zweiten Druckelements 250 und der Sicherungsschraube 260 in die Ausnehmung 8 des Schraubenelements 1 eingegriffen wird. Anschließend kann die Winkelstellung der Platte 200 zum Schaft 2 eingestellt werden und durch Anziehen der Sicherungsschraube 260 Druck auf das zweite Druckelement 250 und damit auf das zweite Kopfteil 10 ausgeübt werden, um ein Fixieren der Ruhewinkelstellung zu erzielen.

Dritte Ausführungsform

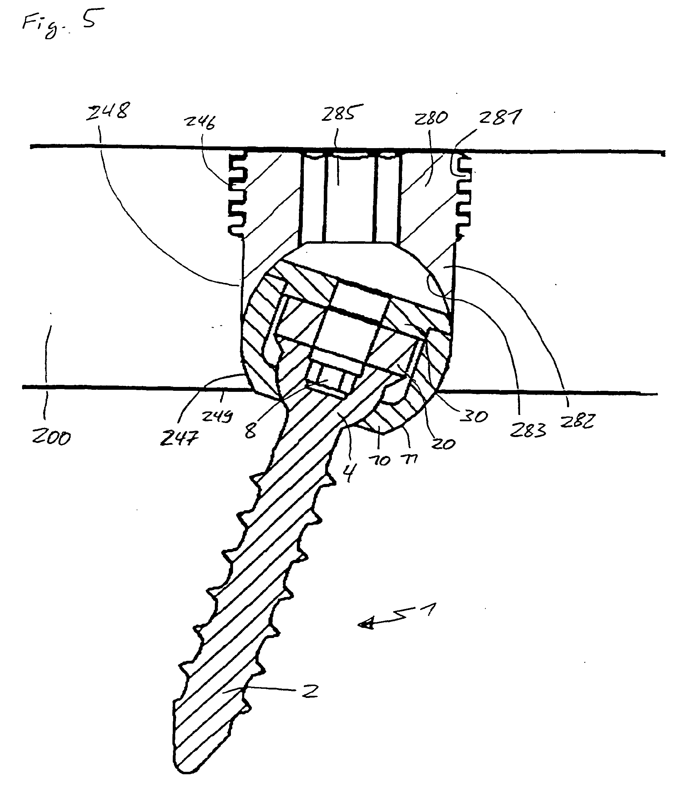

Bei einem Knochenverankerungselement der dritten Ausführungsform, das in Fig. 5 dargestellt ist, unterscheidet sich das Knochenverankerungselement nur dadurch von der zweiten Ausführungsform, dass das zweite Druckelement und die Sicherungsschraube einstückig in Form einer Sicherungsschraube 280 ausgebildet sind.

Bei dieser Ausführungsform entspricht der Aufbau von erstem Schraubenelement 1, erstem Druckelement 20, Befestigungskappe 30, zweitem Kopfteil 10 und Platte 200 dem Aufbau der zweiten Ausführungsform, weshalb für diese Komponenten dieselben Bezugszeichen verwendet werden und eine erneute Beschreibung unterbleibt.

Die Sicherungsschraube 280 weist einen ersten Abschnitt 281 auf, der mit einem Außengewinde 281 zum Einschrauben in das Innengewinde 246 der Bohrung 248 versehen ist. Angrenzend an den ersten Abschnitt 281 weist die Sicherungsschraube 280 auf der Seite, die der zweiten Bohrung 247 zugewandt ist, einen zweiten Abschnitt 282 auf. Der zweite Abschnitt 282 ist im Wesentlichen zylindersymmetrisch mit einem Außendurchmesser, der kleiner ist, als der Innendurchmesser der ersten Bohrung 248, sodass die Sicherungsschraube 280 in die erste Bohrung 248 einschraubbar ist.

Die dem zweiten Kopfteil 10 zugewandte Stirnseite 283 der Sicherungsschraube 280 ist konkav sphärisch ausgebildet, wobei der Radius dem Radius der sphärischen Außenfläche 11 des zweiten Kopfteils 10 und der daran befestigten Befestigungskappe 30 entspricht.

In Richtung der Bohrung in der Platte ist die Höhe der Sicherungsschraube 280 gerade so gewählt, dass durch Anziehen die Stirnfläche 283 derart Druck auf das zweite Kopfteil 10 ausübt, dass es in der Platte in einer zu dem Zeitpunkt des Anziehens der Sicherungsschraube 280 eingestellten Winkelstellung fixierbar ist, die nachfolgend als Ruhewinkelstellung bezeichnet ist. Die Platte 200 wird dabei gleichzeitig mit dem Schraubenelement 1 verbunden.

Mit dieser Anordnung wird erreicht, dass das zweite Kopfteil 10 relativ zu der Platte 200 ausgerichtet und anschließend in dieser Ausrichtung fixiert werden kann.

Die Sicherungsschraube 280 weist eine koaxiale Bohrung 285 auf, die ein Eingreifen mit einem Schraubwerkzeug ermöglicht. In Fig. 5 ist eine sechskantförmige Bohrung 285 für einen Inbusschlüssel dargestellt. Es ist jedoch auch eine andere Formgebung zum Eingreifen mit einem Einschraubwerkzeug möglich.

Die koaxiale Bohrung 285 weist einen solchen Querschnitt auf, dass ein Hindurchgreifen mit dem Schraubwerkzeug, für das die Ausnehmung 8 des ersten Kopfteils vorgesehen ist, ermöglicht ist.

Diese Ausführungsform weist den besonderen Vorteil auf, dass der gesamte Aufbau vormontiert werden kann. Im Betrieb ist zuerst nur die Sicherungsschraube 280 nicht angezogen. Das Schraubenelement kann in dem vormontierten Zustand in den Knochen oder Wirbel eingeschraubt werden, indem mit dem Schraubwerkzeug durch die konzentrischen Bohrungen in dem ersten Druckelement 20, der Befestigungskappe 30 und der Sicherungsschraube 280 in die Ausnehmung 8 des Schraubenelements 1 eingegriffen wird. Anschließend kann die Winkelstellung der Platte 200 zum Schaft 2 eingestellt und mit der Sicherungsschraube 280 fixiert werden.

### Vierte Ausführungsform

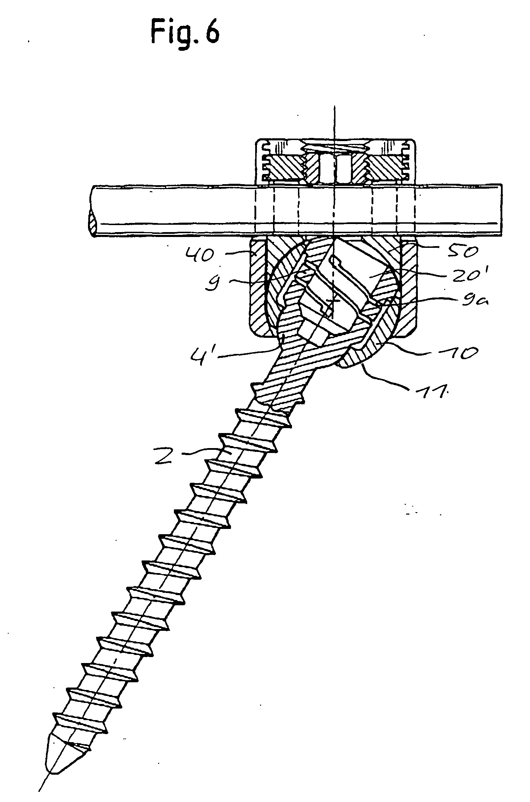

Die in Fig. 6 dargestellte vierte Ausführungsform der vorliegenden Erfindung unterscheidet sich von der ersten bevorzugten Ausführung im Wesentlichen nur in der Ausbildung des ersten Kopfteiles und des elastischen ersten Druckelementes und gleiche oder ähnliche Elemente werden mit denselben Bezugszeichen bezeichnet.

Bei der vierten Ausführungsform schließt sich im Gegensatz zu der ersten Ausführungsform an den verbreiterten Rand 6 auf der dem Schaft 2 abgewandten Seite ein in dieser Ausführungsform mit dem ersten Kopfteil 4' einstückig verbundener zylinderförmiger Abschnitt 9 an, der als elastisches erstes Druckelement dient. Der zylinderförmige Abschnitt 9 erstreckt sich im Wesentlichen in Form eines Hohlzylinders koaxial zu der Achse des Schaftes 2 in Richtung des zweiten Druckelementes 50. In Richtung des zweiten Druckelements 50 weist der zylinderförmige Abschnitt 9 einen Bereich 9b mit einem verbreiterten Außendurchmesser auf, der in der ersten Bohrung 15 des zweiten Kopfteils 10 gehalten ist. Die dem zweiten Druckelement 50 zugewandte Stirnfläche des zylinderförmigen Abschnitts 9 ist derart sphärisch ausgebildet, dass sie im zusammengebauten Zustand des Knochenverankerungselementes eine Fortführung des der sphärischen Außenfläche 11 des zweiten Kopfteiles 10 bildet. Der Zylindermantel des zylinderförmigen Abschnittes 9 weist umlaufende Ausnehmungen 9a derart auf, dass der zylinderförmige Abschnitt 9 eine parallel zu der Schaftachse verlaufende Spiralfeder bildet.

Im Betrieb wird prinzipiell wie bei der ersten Ausführungsform verfahren und das zweite Kopfteil 10 durch den von dem zweiten Druckelement 50 ausgeübten Druck in dem Aufnahmeteil 40 in einer gewünschten Ruhewinkelstellung fixiert. In diesem Zustand ist wiederum, wie bei der ersten Ausführungsform eine Auslenkung des Schaftes 2 relativ zu dem zweiten Kopfteil 10 aus der Ruhewinkelstellung möglich. Bei der Auslenkung aus der Ruhewinkelstellung wird die Spiralfeder des zylinderförmigen Abschnitts 9 elastisch deformiert und es wirkt folglich auf das erste Kopfteil 4' eine in die Ruhewinkelstellung rücktreibende Kraft, wobei bei dieser Ausbildung in Form einer einstückig mit dem ersten Kopfteil 4 ausgebildeten Schraubenfeder eine Druck- und eine Zugseite zu der Rückstellung beitragen, da bei einer Auslenkung eine Seite komprimiert und die gegenüberliegende Seite gedehnt wird.

Durch die Spiralfeder des zylinderförmigen Abschnitts 9 können durch eine geeignete Wahl der Federkraft und der Vorspannung verschieden starke Rücktreibekräfte realisiert werden, was zu einer großen Variabilität des Knochenverankerungselementes führt.

Weiterhin ist auch eine Kombination der Merkmale der vierten Ausführungsführungsform mit denen der anderen Ausführungsformen möglich, insbesondere kann der beschriebene Aufbau auch in einer Platte wie bei der zweiten und dritten Ausführungsform Verwendung finden.

### Weitere Ausführungsformen und Abwandlungen

Das erste Kopfteil 4 und die zweite Bohrung 16 in dem zweiten Kopfteil 10 müssen nicht kugelsegmentförmig bzw. hohlkugelsegmentförmig ausgebildet sein, sondern können eine andere Form aufweisen, solange die gewährleistet ist, daß das erste Kopfteil 4 in dem zweiten Kopfteil schwenkbar gehalten ist.

Ebenfalls müssen das zweite Kopfteil 10 und der das zweite Kopfteil haltende Bereich des Aufnahmeteils nicht kugelsegmentförmig bzw. hohlkugelsegmentförmig sein, sondern müssen ebenfalls eine Schwenkbarkeit des zweiten Kopfteils 10 in dem Aufnahmeteil und bei Fixierung in der gewünschten Ruhewinkelstellung eine ausreichende Haltekraft ermöglichen.

Das Halten der Befestigungskappe 30 in dem zweiten Kopfteil 10 kann auch durch ein auf dem Abschnitt 32 vorgesehenes Außengewinde realisiert sein, das mit einem Innengewinde der Bohrung 15 in einem an das erste Ende 12 angrenzenden Bereich zusammenwirkt.

Das erste Druckelement 20 muss nicht unbedingt aus einem Elastomer bestehen, sondern kann beispielsweise auch durch entsprechend angeordnete Federn realisiert werden, die die Rückstellkraft ausüben.

Es ist ebenfalls denkbar, zum Fixieren des Stabes 100 und zum Fixieren der Ruheposition nur eine Innenschraube zu verwenden, die über den Stab 100 Druck auf das zweite Druckelement 50 und damit auch auf das zweite Kopfteil 10 ausübt.

Der Schaft 2 und das erste Kopfteil 4 sind einstückig ausgebildet gezeigt, jedoch ist auch eine mehrstückige Ausbildung möglich, bei der zum Beispiel Schaft 2 und erstes Kopfteil 4 mittels einer Schraubverbindung, Passsitz oder ähnlichem verbunden sind.

Weitere Abwandlungen der vorher beschriebenen Ausführungsformen sind möglich. Insbesondere können Elemente einer Ausführungsform mit den Elementen einer anderen Ausführungsform kombiniert werden.

Es können zwei oder mehr erfindungsgemäße Knochenverankerungselemente in Kombination mit einem Stab oder mit einer Platte verwendet werden, ebenso können erfindungsgemäße Knochenverankerungselemente mit bekannten Knochenverankerungselementen, insbesondere mit Knochenschrauben oder Haken, kombiniert werden.

Die Erfindung ist nicht auf die konkret beschriebenen Beispiele eines polyaxialen Knochenverankerungselements beschränkt. Es können auch andere Ausbildungen desselben, insbesondere der Aufnahmeteile und der Befestigungseinrichtungen möglich sein. Entscheidend ist, dass der Schaft elastisch aus einer Ruhewinkelstellung auslenkbar ist und dass die Einstellung der Ruhewinkelstellung von der elastischen Auslenkbarkeit entkoppelt ist.

Bei allen vorgenannten Ausführungsformen besteht ferner der Vorteil, daß die begrenzte Bewegungsmöglichkeit der Knochenteile bzw. Wirbel zu einer erhöhten zyklischen Teilbelastung führt, die das Knochenwachstum anregt. Gleichzeitig bleibt die Sicherheit der Verankerung im Knochen gewährleistet.

## Patentansprüche

1. Knochenverankerungselement mit
einem Element (1) mit einem Schaft (2) zur Verankerung in einem Knochen oder einem Wirbel und mit einem mit dem Schaft (2) verbundenen ersten Kopfteil (4),
einem mit dem Element (1) verbindbaren zweiten Kopfteil (10), in dem das erste Kopfteil schwenkbar gehalten ist,
einem Aufnahmeteil (40, 200), in dem das zweite Kopfteil (10) aufgenommen ist, und
einer Fixiervorrichtung (60; 260; 280) zum Fixieren des zweiten Kopfteils (10) in dem Aufnahmeteil (40; 200)
**dadurch gekennzeichnet, dass**
das zweite Kopfteil (10) hülsenförmig zum Aufnehmen des ersten Kopfteils (4) ausgebildet und in dem Aufnahmeteil (40, 200) schwenkbar gehalten ist,
die Fixiervorrichtung das zweite Kopfteil (10) in dem Aufnahmeteil (40, 200) in einer Stellung fixiert, die eine Ruhewinkelstellung des Schaftes (2) relativ zu dem Aufnahmeteil bestimmt, und
das erste und das zweite Kopfteil derart miteinander elastisch verbunden sind, dass der Schaft (2) relativ zu dem zweiten Kopfteil (10) aus der Ruhewinkelstellung auslenkbar ist und dabei auf das erste Kopfteil (4) eine in die Ruhewinkelstellung rücktreibende Kraft wirkt.

2. Knochenverankerungselement nach Anspruch 1, **dadurch gekennzeichnet, dass** die Auslenkung des Schaftes (2) aus der Ruhewinkelstellung von dem Fixieren der Ruhewinkelstellung entkoppelt ist.

3. Knochenverankerungselement nach Anspruch 1 oder 2, die derart ausgebildet ist, dass ein elastisches Druckelement (20, 9) vorgesehen ist, das auf das erste Kopfteil (4) einwirkt.

4. Knochenverankerungselement nach Anspruch 3, **dadurch gekennzeichnet, dass** das elastische Druckelement (20) aus einem Elastomer ausgebildet ist.

5. Knochenverankerungselement nach Anspruch 3, **dadurch gekennzeichnet, dass** das elastische Druckelement (9) einstückig mit dem ersten Kopfteil (4) ausgebildet ist.

6. Knochenverankerungselement nach einem der Ansprüche 1 bis 5, **dadurch gekennzeichnet, dass** das zweite Kopfteil (10) eine im Wesentlichen kugelsegmentförmige Außenfläche aufweist.

7. Knochenverankerungselement nach einem der Ansprüche 1 bis 6, **dadurch gekennzeichnet, dass** das erste Kopfteil (4) eine im Wesentlichen kugelsegmentförmige Form aufweist.

8. Knochenverankerungselement nach einem der Ansprüche 1 bis 7, **dadurch gekennzeichnet, dass** der Innendurchmesser des hülsenförmigen zweiten Kopfteils (10) in einem ersten Bereich (15) größer ist als der Durchmesser des ersten Kopfteils (4) und in einem zweiten Bereich (16) kleiner ist, so dass das erste Kopfteil (4) darin einführbar und schwenkbar gehalten ist.

9. Knochenverankerungselement nach einem der Ansprüche 1 bis 8, **dadurch gekennzeichnet, dass** das erste Kopfteil (4) an seiner dem Schaft (2) abgewandten Seite einen verbreiterten Rand (6) aufweist.

10. Knochenverankerungselement nach Anspruch 9, **dadurch gekennzeichnet, dass** der verbreiterte Rand (6) die Auslenkbarkeit des ersten Kopfteils (4) aus der Ruhewinkelstellung beschränkt.

11. Knochenverankerungselement nach einem der Ansprüche 3 bis 10, **dadurch gekennzeichnet, dass** das elastische Druckelement (20, 9) unter Vorspannung zwischen dem ersten Kopfteil (4) und einer das zweite Kopfteil (10) bedeckenden Kappe (30) befestigt ist.

12. Knochenverankerungselement nach einem der Ansprüche 1 bis 11, **dadurch gekennzeichnet, dass** ein zweites Druckelement (50; 250; 280) vorgesehen ist, das zum Fixieren der Ruhewinkelstellung auf das zweite Kopfteil einwirkt.

13. Knochenverankerungselement nach einem der Ansprüche 1 bis 12, **dadurch gekennzeichnet, dass** das Aufnahmeteil (40) im wesentlichen hohlzylinderförmig ausgebildet ist und an einem Ende einen Bereich (47) mit einem verjüngten Innendurchmesser zum Halten des zweiten Kopfteils (10) aufweist.

14. Knochenverankerungselement nach einem der Ansprüche 1 bis 12, **dadurch gekennzeichnet, dass** das Aufnahmeteil als eine Platte (200) mit einer im wesentlichen hohlzylinderförmigen Bohrung (248; 247) ausgebildet ist und an einem Ende der Bohrung einen Bereich (247) mit einem verjüngten Innendurchmesser zum Halten des zweiten Kopfteils (10) aufweist.

15. Stabilisierungseinrichtung mit einem ersten Knochenverankerungselement und einem zweiten Knochenverankerungselement und einem diese verbindenden Stab (100) oder einer Platte (200), **dadurch gekennzeichnet, dass** wenigstens eines der Knochenverankerungselemente nach einem der Ansprüche 1 bis 14 ausgebildet ist.

## Claims

1. Bone anchoring element with
an element (1) having a shaft (2) for anchoring in a bone or vertebra, and having a first head part (4) connected to the shaft (2),
a second head part (10) connectable to the element (1), in which the first head part is pivotably held,
a receiving part (40; 200) in which the second head part (10) is received, and
a fixation device (60; 260; 280) for fixing the second head part (10) in the receiving part (40; 200),
**characterized in that**
the second head part (10) is formed as sleeve-shaped for receiving the first head part (4) and is pivotably held in the receiving part (40, 200),
the fixing device fixes the second head part (10) in the receiving part (40, 200) in a position which determines a resting angular position of the shaft (2) relative to the receiving part, and
the first and second head parts are elastically connected to each other such that the shaft (2) is deflectable from the resting angular position relative to the second head part (10) and, in doing so, a force restoring to the resting angular position acts on the first head part (4).

2. Bone anchoring element according to Claim 1, **characterized in that** the deflection of the shaft (2) from the resting angular position is decoupled from the fixation of the resting angular position.

3. Bone anchoring element according to Claim 1 or 2, being formed such that an elastic pressure element (20, 9) acting on the first head part (4) is provided.

4. Bone anchoring element according to Claim 3, **characterized in that** the elastic pressure element (20) is made from an elastomer.

5. Bone anchoring element according to Claim 3, **characterized in that** the elastic pressure element (9) is formed in one piece with the first head part (4).

6. Bone anchoring element according to any one of Claims 1 to 5, **characterized in that** the second head part (10) comprises an outer surface essentially shaped as a segment of a sphere.

7. Bone anchoring element according to any one of Claims 1 to 6, **characterized in that** the first head part (4) essentially has a shape as a segment of a sphere.

8. Bone anchoring element according to any one of Claims 1 to 7, **characterized in that**, in a first region (15), the inner diameter of the sleeve-shaped second head part (10) is larger than the diameter of the first head part (4) and, in a second region (16), is smaller, such that the first head part (4) is insertable therein and pivotably held.

9. Bone anchoring element according to any one of Claims 1 to 8, **characterized in that** the first head part (4) comprises a widened edge (6) at its side facing away from the shaft (2).

10. Bone anchoring element according to Claim 9, **characterized in that** the widened edge (6) limits the capability of deflection of the first head part (4) from the resting angular position.

11. Bone anchoring element according to any one of Claims 3 to 10, **characterized in that** the elastic pressure element (20, 9) is attached under pre-load between the first head part (4) and a cap (30) covering the second head part (10).

12. Bone anchoring element according to any one of Claims 1 to 11, **characterized in that** a second pressure element (50; 250; 280) is provided which, for fixing the resting angular position, acts on the second head part.

13. Bone anchoring element according to any one of Claims 1 to 12, **characterized in that** the receiving part (40) is provided to be essentially hollow cylindrical in shape and, at one end, comprises a region (47) with a tapered inner diameter for holding the second head part (10).

14. Bone anchoring element according to any one of Claims 1 to 12, **characterized in that** the receiving part is provided as a plate (200) with an essentially hollow cylindrical bore (248; 247) and, at one end of the bore, comprises a region (247) with a tapered inner diameter for holding the second head part (10).

15. Stabilization device with a first bone anchoring element and a second bone anchoring element and a rod (100) or a plate (200) connecting these, **characterized in that** at least one of the bone anchoring elements is provided according to any one of Claims 1 to 14.

## Revendications

1. Élément d'ancrage osseux, comprenant :
un élément (1) avec une tige (2), pour ancrage dans un os ou une vertèbre, et avec une première partie de tête (4) reliée à la tige (2),
une deuxième partie de tête (10), susceptible d'être reliée à l'élément (1) et dans laquelle la première partie de tête est maintenue de façon à pouvoir pivoter,
une partie de logement (40, 200) dans laquelle la deuxième partie de tête (10) est logée, et
un dispositif de fixation (60; 260; 280) pour fixation de la deuxième partie de tête (10) dans la partie de logement (40 ; 200),
**caractérisé en ce que**
la deuxième partie de tête (10) est réalisée en forme de douille pour recevoir la première partie de tête (4) et est maintenue à pivotement dans la partie de logement (40 ; 200),
le dispositif de fixation fixe la deuxième partie de tête (10) dans la partie de logement (40 ; 200), en une position déterminant une position angulaire de repos de la tige (2) par rapport à la partie de logement, et
la première et la deuxième partie de tête étant reliées ensemble de façon élastique, de manière que la tige (2) puisse être déviée par rapport à la deuxième partie de tête (10) en partant d'une position angulaire de repos, et qu'une force, ramenant la première partie de tête (4) à la position angulaire de repos, agisse.

2. Élément d'ancrage osseux selon la revendication 1, **caractérisé en ce que** le débattement de la tige (2) depuis la position de repos, est désaccouplé de la fixation de la position angulaire de repos.

3. Élément d'ancrage osseux selon la revendication 1 ou 2, réalisé de manière qu'un élément de pressage (20, 9) élastique soit prévu, agissant sur la première partie de tête (4).

4. Élément d'ancrage osseux selon la revendication 3, **caractérisé en ce que** l'élément de pressage (20) élastique est réalisé en un élastomère.

5. Élément d'ancrage osseux selon la revendication 3, **caractérisé en ce que** l'élément de pressage (9) élastique est réalisé d'une seule pièce avec la première partie de tête (4).

6. Élément d'ancrage osseux selon l'une des revendications 1 à 5, **caractérisé en ce que** la deuxième partie de tête (10) présente une surface extérieure sensiblement en forme de segment de sphère.

7. Élément d'ancrage osseux selon l'une des revendications 1 à 6, **caractérisé en ce que** la première partie de tête (4) présente une forme sensiblement en forme de segment de sphère.

8. Élément d'ancrage osseux selon l'une des revendications 1 à 7, **caractérisé en ce que** le diamètre intérieur de la deuxième partie de tête (10) en forme de douille, dans une première zone (15), est plus grand que le diamètre de la première partie de tête (4) et, dans une deuxième zone (16), est plus petit, de manière que la première partie de tête (4) puisse y être introduite et y être maintenue à pivotement.

9. Élément d'ancrage osseux selon l'une des revendications 1 à 8, **caractérisé en ce que** la première partie de tête (4) présente sur sa face opposée à la tige (2) un bord (6) élargi.

10. Élément d'ancrage osseux selon la revendication 9, **caractérisé en ce que** le bord (6) élargi limite la possibilité de débattement de la première partie de tête (4) depuis la position angulaire de repos.

11. Élément d'ancrage osseux selon l'une des revendications 3 à 10, **caractérisé en ce que** l'élément de pressage (20, 9) élastique est fixé sous précontrainte entre la première partie de tête (4) et un capuchon (30) couvrant la deuxième partie de tête (10).

12. Élément d'ancrage osseux selon l'une des revendications 1 à 11, **caractérisé en ce qu'**un deuxième élément de pressage (50 ; 250 ; 280) est prévu, agissant pour fixer la position angulaire de repos sur la deuxième partie de tête.

13. Élément d'ancrage osseux selon l'une des revendications 1 à 12, **caractérisé en ce que** la partie de logement (40) est réalisée sensiblement avec une forme de cylindre creux, et présente à une extrémité une zone (47) ayant un diamètre intérieur effilé pour maintenir la deuxième partie de tête (10).

14. Élément d'ancrage osseux selon l'une des revendications 1 à 12, **caractérisé en ce que** la partie de logement est réalisée sous la forme d'une plaque (200) ayant un perçage (248 247) sensiblement en forme de cylindre creux, et présente sur une extrémité du perçage une zone (247) ayant un diamètre intérieur effilé, pour assure le maintien de la deuxième partie de tête (10).

15. Dispositif de stabilisation comprenant un premier élément d'ancrage osseux et un deuxième élément d'ancrage osseux, et une barre (100) ou une plaque (200) reliant ceux-ci, **caractérisé en ce qu'**au moins l'un des éléments d'ancrage osseux est réalisé selon l'une des revendications 1 à 14.
